# EUROPÄISCHE PATENTSCHRIFT

(11) **EP 0 502 936 B1**
(45) Veröffentlichungstag und Bekanntmachung des Hinweises auf die Patenterteilung: **28.07.1993**
(21) Anmeldenummer: 91900238.6
(22) Anmeldetag: 26.11.1990
(51) Int. Cl.: A61K 31/47, A61K 9/08

(54) **MITONAFIDE-INFUSIONSLÖSUNG**
MITONAPHIDE INFUSION SOLUTION
INFUSION DE MITONAFIDE

(30) Priorität: 29.11.1989 DE 3939372
(43) Veröffentlichungstag der Anmeldung: 16.09.1992
(73) Patentinhaber: KNOLL AG, D-67061 Ludwigshafen (DE)
(72) Erfinder: TETZNER, Christine, D-6700 Ludwigshafen (DE); MARTIN JIMENEZ, Miguel, E-28028 Madrid (ES); ROSELL COSTA, Rafael, E-8034 Barcelona (ES)
(74) Vertreter: Karau, Wolfgang, Dr.
(86) Internationale Anmeldenummer: EP9002023
(87) Internationale Veröffentlichungsnummer: WO9107965

(56) Entgegenhaltungen:
- EP-A- 0 268 093
- EP-A- 0 268 100
- DE-A- 3 707 652
- European Journal of Drug Metabolism and Pharmacokinetics, vol. 11, no. 4, 1986, P. Rivera Cid et al.

## Beschreibung

Mitonafide (N-(2-Dimethylaminoethyl)-3-nitronaphthalsäureamid, DE 2 323 555) ist eine antineoplastisch wirkende Substanz, die interkalativ wirkt. Die Substanz wird in der Regel an 5 aufeinanderfolgenden Tagen dem Patienten als Bolus-Injektion appliziert. 3 Wochen nach einer Applikation wird die Gabe wiederholt. Dabei zeigt sich, daß nach einer täglichen Bolus-Gabe von ab etwa 100 mg pro m² Körperoberfläche unerwünschte Wirkungen auf das Zentralnervensystem auftreten, wie Desorientiertheit und partieller Gedächtnisschwund.

Es wurde nun gefunden, daß diese Nebenwirkungen nicht auftreten, wenn man Mitonafide infundiert.

Gegenstand der Erfindung ist eine Infusionslösung, die Mitonafide enthält.

Die Konzentration von Mitonafide in der Lösung muß so gewählt werden, daß die dem Patienten infundierte Tagesmenge bei 150 bis 300 mg pro m² Körperoberfläche liegt. Bei einer täglichen kontinuierlich infundierten Menge von bis zu 5 l sind das etwa 50 - 150 mg Mitonafide pro 1.

Zur Infusion wird das Mitonafide zweckmäßig einer Infusionslösung zugegeben. Bevorzugt sind Infusionslösungen zur Elektrolytzufuhr. (Rote Liste 1988, 51 001 - 51 092) oder Lösungen zum Volumenersatz (Rote Liste 1988, 51 319 - 51 356). Mitonafide kann auch mit Lösungen zur Zufuhr von Aminosäuren appliziert werden. (Rote Liste 1988, 51 218 -51 270). Die Lösungen sollen jedoch keine reduzierenden Bestandteile wie z. B. Zucker enthalten. Nach der Zugabe von Mitonafide zur Infusionslösung muß der pH-wert kontrolliert und gegebenenfalls neu eingestellt werden. Er sollte bei 4 bis 7, vorzugsweise 5,5 bis 6,5 liegen.

Infundiert man einem Patienten über einen Zeitraum von 6 bis 24 Stunden täglich eine Menge von 150 bis 300 mg Mitonafide pro m² Körperoberfläche, so werden im Gegensatz zur schnellen intravenösen Applikation (Bolus) überraschenderweise keine Nebenwirkungen auf das Zentralnervensystem beobachtet. Die Infusionslösung eröffnet daher die Möglichkeit, Neoplasmen gezielter zu bekämpfen. Das gilt insbesondere für nicht kleinzellige Lungenkarzinome sowie Mammakarzinome.

### Beispiel

In 15 l physiologischer Kochsalzlösung werden 3,6 g Mitonafide gegeben und der pH-wert auf pH 6,0 eingestellt. Die Lösung wird über Sterilfilter in dreißig 0,5 l-Flaschen gefüllt. Die Flaschen werden anschließend in üblicher weise verschlossen und sterilisiert.

## Patentansprüche

1. Infusionslösung, enthaltend Mitonafide.

2. Verfahren zur Herstellung einer Mitonafide-Infusionslösung, dadurch gekennzeichnet, daß man Mitonafide einer bekannten Infusionslösung zusetzt.

## Claims

1. An infusion solution containing mitonafide.

2. A process for preparing a mitonafide infusion solution, which comprises adding mitonafide to a known infusion solution.

## Revendications

1. Infusion contenant du mitonafide.

2. Procédé de préparation d'une infusion de mitonafide, caractérisé par le fait que l'on ajoute du mitonafide à une infusion connue.
